# EUROPÄISCHE PATENTSCHRIFT

(11) **EP 1 244 776 B1**
(45) Veröffentlichungstag und Bekanntmachung des Hinweises auf die Patenterteilung: **06.04.2005**
(21) Anmeldenummer: 00988619.3
(22) Anmeldetag: 17.11.2000
(51) Int. Cl.: C12N 9/02, C12N 15/52, C12N 15/62, C12N 15/82, C12N 5/10, C12Q 1/68

(54) **TETRAHYDROPYRIMIDIN-DIOXYGENASE-GEN, DADURCH KODIERTE POLYPEPTIDE UND VERFAHREN ZUR DEREN HERSTELLUNG**
TETRAHYDROPYRIMIDINE OXYGENASE GENE, POLYPEPTIDES ENCODED BY SAID GENE AND METHOD FOR PRODUCING THE SAME
GENE DE TETRAHYDROPYRIMIDINE DIOXYGENASE, POLYPEPTIDES CODES PAR CES GENES, ET PROCEDES DE FABRICATION DE CES POLYPEPTIDES

(30) Priorität: 24.11.1999 DE 19957470
(43) Veröffentlichungstag der Anmeldung: 02.10.2002
(73) Patentinhaber: MERCK PATENT GmbH, 64293 Darmstadt (DE)
(72) Erfinder: KELLER, Ullrich, 14890 Berlin (DE); GRAMMEL, Nicolas, 12249 Berlin (DE)
(74) Vertreter: Nobbe, Matthias, Dr.
(86) Internationale Anmeldenummer: PCT/DE2000/004036
(87) Internationale Veröffentlichungsnummer: WO 2001/038500

(56) Entgegenhaltungen:
- MALIN GENNADY ET AL: "Induction of synthesis of tetrahydropyrimidine derivatives in Streptomyces strains and their effect on Escherichia coli in response to osmotic and heat stress." JOURNAL OF BACTERIOLOGY, Bd. 178, Nr. 2, 1996, Seiten 385-395, XP000996665 ISSN: 0021-9193 in der Anmeldung erwähnt
- ZAMRI ADEL ET AL: "A stereocontrolled synthesis of a new class of 3,4,5,6-tetrahydropyrimidine-based chiral amino acids." TETRAHEDRON, Bd. 55, Nr. 16, 16. April 1999 (1999-04-16), Seiten 5157-5170, XP004161094 ISSN: 0040-4020
- DATABASE NAGENESEQ [Online] Accession No. T47925, 18. Juni 1997 (1997-06-18) "DNA encoding L-proline-4-hydroxylase" XP002166790

### Sequenzprotokoll

<110> Grammel, Nicholas, Dr. Keller, Ulrich, Dr.
<120> Tetrahydroxypyrimidin-Dioxygenase-Gen, Polypeptide mit Tetrahydropyrimidin-Dioxygenase-Aktivität, Verfahren zur Herstellung dieser Polypeptide sowie deren Verwendung zur Produktion von hydroxyliertem Tetrahydropyrimidin
<140> DE 199 57 470.7
   <141> 1999-11-24
<160> 6
<210> 1
   <211> 979
   <212> DNA
   <213> Streptomyces chrysomallus
<400> 1
<210> 2
   <211> 30
   <212> DNA
   <213> künstliche Sequenz
<220>
   <223> Als PCR-Primer verwendet, stammend aus dem Plasmid pQEthpD. Das Plasmid pQEthpD ist das Plasmid pQE30, welches das thpD Gen als BamHI/HindIII PCR-Fragment enthält.
<400> 2
   gcctgaggat ccatgaccac cgaagtacgc                                          30
<210> 3
   <211> 27
   <212> DNA
   <213> künstliche Sequenz
<220>
   <223> Als PCR-Primer verwendet, stammend aus dem Plasmid pQEthpD. Das Plasmid pQEthpD ist das Plasmid pQE30, welches das thpD Gen als BamHI/HindIII PCR-Fragment enthält.
<400> 3
   cccgcccgtg aagcttgcta cttcacc 27
<210> 4
   <211> 30
   <212> DNA
   <213> künstliche Sequenz
<220>
   <223> Als PCR-Primer verwendet, stammend aus dem Plasmid pQEthpD. Das Plasmid pQEthpD ist das Plasmid pQE30, welches das thpD Gen als BamHI/HindIII PCR-Fragment enthält.
<400> 4
   gaggaggaat tcagcatgcg aggatcgcat 30
<210> 5
   <211> 25
   <212> DNA
   <213> künstliche Sequenz
<220>
   <223> Als PCR-Primer verwendet, stammend aus dem Plasmid pQEthpD. Das Plasmid pQEthpD ist das Plasmid pQE30, welches das thpD Gen als BamHI/HindIII PCR-Fragment enthält.
<400> 5
   agtccaagct cagctaatta agctt 25
<210> 6
   <211> 297
   <212> PRT
   <213> Streptomyces chrysomallus
<400> 6
   Met Thr Thr Glu Val Arg Ala Asp Leu Tyr Pro Ser
    1               5                  10
   Arg Gly Ala Ala Glu Met Thr Thr Pro Arg Gln Asp Pro Val Ile Trp Ser
           15                  20                  25
   Ala Pro Gly Ala Pro Gly Pro Val Ala Ala Lys Asp Leu Gln Gly Tyr Glu
    30                  35                  40                  45
   His Asp Gly Phe Leu Thr Val Asp Gln Leu Ile Ala Pro Asp Glu Val Ala
               50                  55                  60
   Val Tyr Gln Ala Glu Leu Asn Arg Leu Ile Ser Asp Pro Ala Val Arg Ala
       65                  70                  75                  80
   Asp Glu Arg Ser Ile Val Glu Lys Gln Ser Gln Asn Val Arg Ser Val Phe
                   85                  90                  95
   Glu Val His Arg Ile Ser Glu Val Phe Ala Glu Leu Val Arg Asp Glu Arg
           100                 105                 110
   Val Val Gly Arg Ala Arg Gln Ile Leu Gly Ser Asp Val Tyr Val His Gln
   115                 120                 125                 130
   Ser Arg Ile Asn Val Lys Pro Gly Phe Gly Ala Thr Gly Phe Tyr Trp His
               135                 140                 145
   Ser Asp Phe Glu Thr Trp His Ala Glu Asp Gly Leu Pro Asn Met Arg Thr
       150                 155                 160                 165
   Val Ser Val Ser Ile Ala Leu Thr Glu Asn Phe Asp Thr Asn Gly Gly Leu
                   170                 175                 180
   Met Ile Met Pro Gly Ser His Lys Thr Phe Leu Gly Cys Ala Gly Glu Thr
           185                 190                 195
   Pro Lys Asp Asn Tyr Lys Lys Ser Leu Gln Met Gln Asp Ala Gly Thr Pro
   200                 205                 210                 215
   Ser Asp Glu Ala Leu Thr Lys Met Ala Asp Arg His Gly Ile Arg Leu Phe
               220                 225                 230
   Thr Gly Arg Ala Gly Ser Ala Thr Trp Phe Asp Cys Asn Ala Met His Gly
       235                 240                 245                 250
   Ser Gly Asp Asn Ile Thr Pro Tyr Ala Arg Ser Asn Val Phe Ile Val Phe
                   255                 260                 265
   Asn Ser Val Glu Asn Ala Ala Gln Glu Pro Phe Ala Ala Pro Ile Arg Arg
           270                 275                 280
   Pro Glu Phe Ile Gly Ala Arg Asp Phe Thr Pro Val Lys
   285                 290                 295

## Beschreibung

Die vorliegende Erfindung betrifft Enzyme mit Tetrahydropyrimidin-Dioxygenase-Aktivität, die dafür kodierenden Gene, die homologe und heterologe Expression dieser Gene, Verfahren zur Herstellung von Enzymen mit Tetrahydropyrimidin-Dioxygenase-Aktivität und deren Verwendung dieser Enzyme zur in vivo und in vitro Produktion von hydroxyliertem Tetrahydropyrimidin. Zudem betrifft die Erfindung die biotechnologische Verwendung des nach einem der erfindungsgemässen Verfahren hergestellten hydroxylierten Tetrahydropyrimidins.

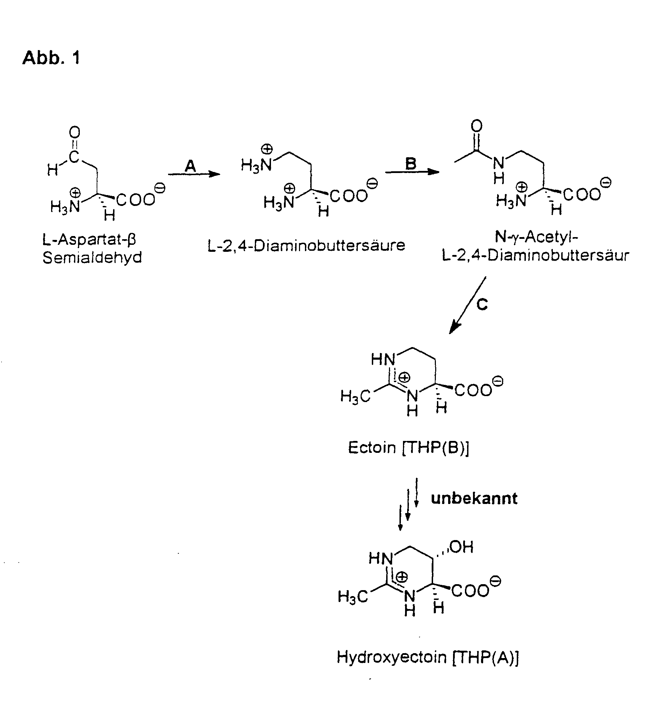

In Folge von osmotischem Stress werden von Mikroorganismen niedermolekulare intrazelluläre Verbindungen gebildet, die als Osmolyte bezeichnet werden und zu denen auch die Tetrahydropyrimidine zählen. Die Tetrahydropyrimidine, THP(B) (2-Methyl-4-carboxy-3,4,5,6-tetrahydropyrimidin) und THP(A) (2-Methyl-4-carboxy-5-hydroxy-3,4,5,6-tetrahydropyrimidin) werden von verschiedenen Mikroorganismen als intrazelluläre Komponenten des Cytoplasmas gebildet [Ventosa, A. et al. (1998). Biology of moderately halophilic aerobic bacteria. *Microbiol. Mol. Biol. Rev.* **62,** 504-544.] (Abb.1). Zu diesen Mikroorganismen gehören die halophilen Eubakterien, Actinomyceten, Bacillusarten und Brevibakterien. Die THPs können aus diesen Organismen in reiner Form isoliert werden und in vielfältiger Form in der Biotechnologie eingesetzt werden, wie z.B. bei der Stabilisierung von Proteinen sowohl in Lösung als auch bei deren Gefriertrocknung [Lippert, K. und Galinski, E.A. (1992). Enzyme stabilisation by ectoine-type compatible solutes: protection against heating, freezing and drying. *Appl. Microbiol. Biotechnol*. **37**, 61-65.], bei der Beeinflussung der Wechselwirkung von Proteinen und Nukleinsäuren [Lapidot, A. et al. (1995). Tetrahydropyrimidine derivatives inhibit binding of a Tat-like, arginine-containing peptide, to HIV TAR RNA in vitro. *FEBS Lett.* **367**, 33-38.; Malin, G. und Lapidot, A. (1996). Induction of synthesis of tetrahydropyrimidine derivatives in *Streptomyces* strains and their effect on *Escherichia coli* in response to osmotic and heat stress. *J. Bacteriol*. **178**, 385-395.; Malin, G. et al. (1999). Effect of tetrahydropyrimidine derivatives on proteinnucleic acids interaction. Type 11 restriction endonucleases as a model system. *J. Biol. Chem*. **274**, 6920-6929.] und bei der Rückfaltung von denaturierten Proteinen. Darüberhinaus kann durch Zugabe von THP(A) bzw. THP(B) zu Kulturmedien von verschiedenen Prokaryonten, wie z.B. *E. coli* eine erhöhte Salz- und Hitzetoleranz festgestellt werden [Malin, G. und Lapidot, A. (1996). Induction of synthesis of tetrahydropyrimidine derivatives in *Streptomyces* strains and their effect on *Escherichia coli* in response to osmotic and heat stress. *J. Bacteriol.* **178,** 385-395.].

Der charakterisierte Biosyntheseweg von THP(A), gezeigt in Abb. 1, startet von dem L-Aspartat-β-semialdehyd. In der nachfolgenden Transaminierungsreaktion katalysiert die L-2,4-Diaminobuttersäure-Transaminase die Bildung von L-2,4-Diaminobuttersäure (DABA). Die nachfolgende N-Acetylierung von DABA erfolgt durch die L-2,4-Diaminobuttersäure-Acetyltransferase unter Verwendung von Acetyl-Coenzym A. Durch die N-Acetyl-(-L,2,4-Diaminobuttersäure-Cyclase katalysierte intramolekulare Kondensationsreaktion entsteht THP(B) [Peters, P. et al. (1990). The biosynthesis of ectoine. *FEMS Microbiol. Lett.* **71,** 157-162. ;, Ono, H. et al. (1999). Characterization of biosynthetic enzymes for ectoine as a compatible solute in a moderately halophilic eubacterium, *Halomonas elongata. J. Bacteriol.* **181,** 91-99.]. Demgegenüber verblieben die Schritte zur Biosynthese des 5-Hydroxyderivates von THP(B), dem (THP(A)) weiterhin ungeklärt.

Die Herstellung von THPs nach den im Stand der Technik bekannten Verfahren ist auf die aufwendige Extraktion dieser Verbindungen aus den Zellen oder Kulturfiltraten von THP(A) und/oder THP(B) produzierenden Bakterien beschränkt. Bedingt durch die hohen Salzkonzentrationen im Kulturmedium sind diese Verfahren besonders kostenintensiv.

Eine chemische Synthese von THPs ist zwar möglich, jedoch umfasst diese Synthese eine Vielzahl von Syntheseschritten und den Einsatz einer aufwendigen Schutzgruppentechnik, so dass dieses komplexe Verfahren, insbesondere im großen Maßstab, nicht wirtschaftlich durchzuführen ist. Dies betrifft im besonderen die Synthese von THP(A), da die jeweiligen Vorstufen auf chemischen Wege nicht erhältlich sind und folglich die chemische Synthese von THP(A) zusätzlich erschwert ist.

Die Aufgabe der Erfindung besteht daher darin, ein verbessertes Verfahren zur Herstellung von hydroxilierteTHPs, insbesondere THP(A) bereitzustellen.

Die Aufgabe der Erfindung wird gelöst durch Bereitstellung eines Verfahrens unter Verwendung eines Polypeptides mit einer Tetrahydropyrimidin-Dioxygenase-Aktivität.

Ein Aspekt der Erfindung betrifft daher Nukleinsäuresequenzen, die für Polypeptide kodieren, die eine Tetrahydropyrimidin-Dioxygenase-Aktivität aufweisen. Diese Nukleinsäuresequenzen umfassen sowohl DNA- als auch RNA-Nukleinsäuresequenzen und sind aus der folgenden Gruppe von Nukleinsäuresequenzen ausgewählt:
(a) DNA-Nukleinsäuresequenz mit der in SEQ. ID. NO.: 1 dargestellten Nukleotidsequenz;
(b) DNA-Nukleinsäuresequenz, die sich als Ergebnis des degenerierten genetischen Codes von der in SEQ. ID. NO.: 1 dargestellten Nukleotidsequenz ableitet;
(c) DNA-Nukleinsäuresequenz, die mit mindestens einer DNA-Nukleinsäuresequenz, die eine DNA-Nukleinsäuresequenz gemäß (a) oder (b) aufweist, hybridisiert;
(d) DNA-Nukleinsäuresequenz, die Fragmente, allelische oder andere Variationen der DNA-Nukleinsäuresequenz mit der in SEQ. ID. NO.: 1 dargestellten Nukleotidsequenz, ist;
(e) RNA-Nukleinsäuresequenz, die von den DNA-Nukleinsäuresequenz gemäß (a) bis (d) abgeleitet ist; und
(f) Nukleinsäuresequenz mit einem Grad der Homologie zu einer der Sequenzen gemäß (a) bis (e) von mehr als 60%.

Mit den für Polypeptide mit Tetrahydropyrimidin-Dioxygenase-Aktivität kodierenden DNA-Nukleinsäuresequenzen sind sowohl genomische DNA-Nukleinsäuresequenzen als auch cDNA-Nukleinsäuresequenzen gemeint. Der Grad der Homologie der Nukleinsäuresequenz gemäß f) zu einer der Sequenzen gemäß (a) bis (e) beträgt mindestens 60%, bevorzugt 80% und besonders bevorzugt mehr als 90%.

Ein weiterer Aspekt der vorliegenden Erfindung betrifft Polypeptide, die eine Tetrahydropyrimidin-Dioxygenase-Aktivität aufweisen und die bevorzugt durch eine der Nukleinsäuresequenzen gemäss (a), (b), (c), (d) (e) oder (f) kodiert sind. Besonders bevorzugt ist ein Polypeptid (SEQ. ID. NO.: 6), das durch die DNA-Nukleinsäuresequenz gemäß SEQ. ID. NO.: 1 kodiert ist.

In einem anderen Aspekt sind DNA- oder RNA-Nukleinsäuresequenzen gemäss (a), (b), (c), (d), (e) oder (f), die aus einem Archaebakterium, einem Prokaryonten oder einem Eukaryonten isoliert wurden, Gegenstand der vorliegenden Erfindung. Besonders bevorzugt sind derartige Nukleinsäuresequenzen, wenn sie aus einem halophilen Eubakterium, einem Actinomyceten, einem Bazillus oder einem Brevibakterium isoliert wurden.

Bevorzugt sind ebenfalls Nukleinsäurekonstrukte, die eine operative Einheit umfassen, welche aus einer Promotersequenz und einer Nukleinsäuresequenz gemäss (a), (b), (c), (d), (e) oder (f) besteht.

Ebenfalls bevorzugt sind Nukleinsäurekonstrukte, die eine operative Einheit umfassen, welche aus einer Promotersequenz und einer Nukleinsäuresequenz gemäss (a), (b), (c), (d), (e) oder (f) besteht und zusätzlich eine Nukleinsäuresequenz umfassen, die für ein oder mehrere Sekretionssignale kodiert.

In einem weiteren Aspekt betrifft die vorliegende Erfindung replikative, rekombinante Vektoren, die eine Nukleinsäuresequenz gemäss (a), (b), (c), (d), (e) oder (f) umfassen.

Gegenstand der Erfindung sind des weiteren Zellen, die einen in dieser Zelle autonom replizierenden Vektor enthalten, der eine Nukleinsäuresequenz gemäss (a), (b), (c), (d), (e) oder (f) umfasst. Besonders bevorzugt sind dabei Bakterienzellen, Hefezellen oder Pflanzenzellen.

Bevorzugt sind Zellen, die eine durch nicht-natürliche Rekombination in das Genom integrierte Nukleinsäuresequenz gemäss (a), (b), (c), (d), (e) oder (f) aufweisen. Besonders bevorzugt sind dabei Zellen, bei denen die nicht-natürliche Rekombination in das Genom einer Bakterienzelle, einer Hefezelle oder einer Pflanzenzelle erfolgt ist.

In einem alternativen Aspekt betrifft die vorliegende Erfindung die Verwendung von einem Oligonukleotid als Nukleinsäure-Sonden zur Identifikation von Genen, wobei die Nukleotidsequenz des Oligonukleotids von jener der Nukleinsäuresequenzen gemäss (a), (b), (c), (d), (e) oder (f) abgeleitet worden sind und die Oligonukleotide zur Identifizierung von sich in in Archaebakterien, Prokaryonten oder Eukaryonten befindlichen, chromosomalen oder extrachromosomalen Genen, die für ein Polypeptid kodieren, welches Tetrahydropyrimidin-Dioxygenase-Aktivität besitzt, verwendet werden.

Gegenstand der Erfindung ist auch eine Pflanze, die mit einer rekombinanten DNA, die eine DNA-Sequenz gemäß einem der Ansprüche 1 bis 4 umfasst, transformiert wurde. Eine solche Pflanze zeichnet sich durch eine gesteigerte osmotische Toleranz aus.

Gegenstand der Erfindung ist des weiteren ein Verfahren zur Herstellung eines Polypeptids mit Tetrahydropyrimidin-Dioxygenase-Aktivität, wobei hierzu Zellen, die einen Nukleinsäureabschnitt mit einer Nukleinsäuresequenz gemäss (a), (b), (c), (d), (e) oder (f) oder ein Nukleinsäurenkonstrukt der oben erläuterten Art enthalten, kultiviert werden und das Polypeptid mit Tetrahydropyrimidin-Dioxygenase-Aktivität isoliert wird.

Die für das Isolieren des Polypeptids verwendete Methode wird in einer dem Fachmann bekannten Weise davon abhängen, ob die zur Herstellung verwendeten Zellen einen Nukleinsäureabschnitt mit einer Nukleinsäuresequenz gemäss (a), (b), (c), (d), (e) oder (f) oder ein Nukleinsäurekonstrukt der oben erläuterten Art enthalten. Bei der ersten Möglichkeit kann ein Isolieren des hergestellten Polypeptids erst nach vorgeschaltetem Aufschluß der Zellen erfolgen, wobei bei der zweiten Möglichkeit, bei der die Zellen ein ein Sekretionssignal umfassendes Nukleinsäurenkonstrukt enthalten, das hergestellte Polypeptid direkt aus dem Kulturmedium isolierbar wäre.

Ein anderer Aspekt der Erfindung betrifft die Verwendung eines Polypeptids mit Tetrahydropyrimidin-Dioxygenase-Aktivität zur Herstellung von hydroxyliertem Tetrahydropyrimidin.

Bevorzugt erfolgt die Herstellung mittels eines Polypeptids mit Tetrahydropyrimidin-Dioxygenase-Aktivität in einer lebenden Zelle, wobei die lebende Zelle eine Bakterienzelle, eine Hefezelle oder eine Pflanzenzelle ist. Die das Polypeptid mit Tetrahydropyrimidin-Dioxygenase-Aktivität enthaltende, lebende Zelle kann sich in einem Kulturmedium befinden, das Tetrahydropyrimidin enthält. In einem solchen Fall ist die lebende Zelle bevorzugt vor der Herstellung zu permeabilisieren, um ein Inkontaktkommen des Polypeptids mit Tetrahydropyrimidin-Dioxygenase-Aktivität mit dem Tetrahydropyrimidin zu begünstigen.

Im folgenden wird eine bevorzugte Ausführungsform der Erfindung beschrieben, wobei diese jedoch nicht in der Weise verstanden werden soll, dass daraus eine Einschränkung des vom Erfinder begehrten Schutzumfangs abgeleitet werden kann.

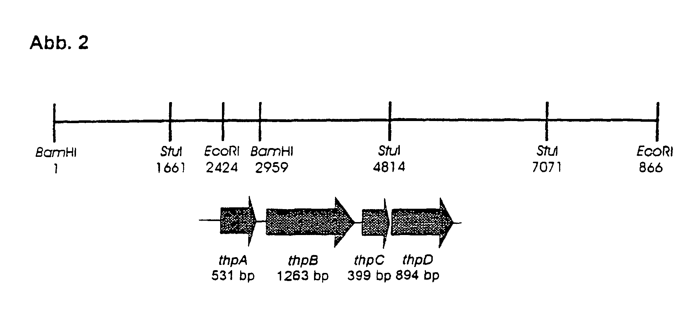

Zur Auffindung der THP-Biosynthesegene in *Streptomyces chrysomallus* wurde die L-2,4-Diaminobuttersäure-Acetyltransferase, ein Enzym der THP-Biosynthese aus *Streptomyces chrysomallus* gereinigt. Von tryptischen Peptidsequenzen der L-2,4-Diaminobuttersäure-Acetyltransferase wurden Oligonukleotidsequenzen abgeleitet und für ein Screening einer Cosmidbank eingesetzt. Das Gen der L-2,4-Diaminobuttersäure-Acetyltransferase sowie flankierende Bereiche wurden sequenziert. Auf dem 8,7 kb großen BamHI/EcoRI Fragment wurden u.a. vier offene Leserahmen gefunden. Diese Gene, die für THP-Biosyntheseenzyme kodieren, werden im folgenden als *thpA, thpB, thpC* und *thpD* bezeichnet (Abb. 2). Der als *thpD* bezeichnete offene Leserahmen kodiert für ein Enzym, das eine THP(B)-Dioxygenase-Aktivität aufweist und ein Molekulargewicht von 32,7 kDa besitzt. Es handelt sich bei diesem Enzym um eine α-Ketoglutarat-abhängige Dioxygenase, die die irreversible Hydroxylierung von THP(B) zu THP(A) katalysiert. Das Gen thpD wurde in *E. coli* und *Streptomyces* exprimiert und das entsprechende Protein (THP(D)) nach im Stand der Technik bekannten Methoden gereinigt.

Die Verwendung des homolog oder heterolog exprimierten THP(D)-Proteins ermöglicht die *in vitro* Produktion von THP(A). Durch Inkubation des THP(D)-Proteins in Gegenwart von THP(B), α-Ketoglutarat, Ascorbinsäure, Eisen(II)sulfat und Katalase wird eine vollständige Hydroxylierung von THP(B) zu THP(A) beobachtet.

Das Gen, das für die Tetrahydropyrimidin-Dioxygenase kodiert, liegt auf einem 8,7 kb großen *BamHI*/*EcoRI* Fragment. Durch die Expression der auf diesem DNA-Fragment enthaltenen Gene aus *Streptomyces chrysomallus* in Mikroorganismen, kann die Synthese von hydroxyliertem Tetrahydropyrimidin erreicht werden.

Durch Expression des Gens *thpD* in Mikroorganismen, die THP(B) produzieren, wird die *in vivo* Produktion des 5-Hydroxyderivates von Tetrahydropyrimidin (THP(A)) ermöglicht.

Dieses Verfahren ist bei einer Vielzahl von Mikroorganismen, wie z.B. Actinomyceten, Bazillen oder halophilen Bakterien anwendbar, sofern diese Nukleinsäuresequenzen besitzen, die für Polypeptide mit THP-Dioxygenase-Aktivität kodieren. Unter dieser Voraussetzung kann die Expression zudem in jedem anderen prokaryontischen oder eukaryontischen Expressionssystem durchgeführt werden.

Im folgenden wird die Erfindung anhand von Beispielen näher beschrieben.

### Beispiel 1: Detektion der L-2,4-Diaminobuttersäure-Acetyltransferase aus Streptomyces chrysomallus

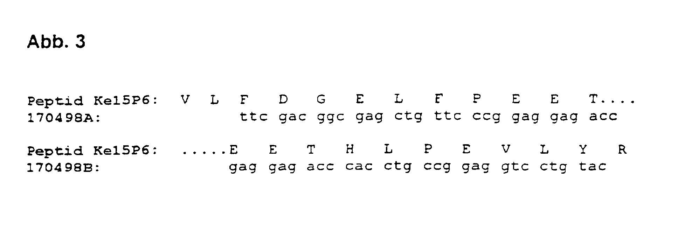

Von einer internalen Peptidsequenz, der aus Streptomyces *chrysomallus* zuvor gereinigten L-2,4-Diaminobuttersäure-Acetyltransferase, wurde eine Nukleotidsequenz abgeleitet (Abb. 3). Diese Nukleotidsequenz wurde radioaktiv markiert und als Sonde zum Screenen einer Cosmidbank eingesetzt. Teile des entsprechenden Cosmids wurden subkloniert und sequenziert. Hierbei wurden u.a. vier offene Leserahmen gefunden, wobei *thpA* für die L-Diaminobuttersäure-Acetyltransferase, *thpB* für die L-Aspartat-ßsemialdehyd-Transaminase, *thpC* für die N-Acetyl-γ-L-2,4-Diaminobuttersäure-Cyclase und *thpD* für die THP(B)-Dioxygenase kodiert (Abb. 2). Die Expression in andere Mikroorganismen ist möglich und als Beispielorganismus dient *Streptomyces lividans* (siehe Beispiel 2).

### Beispiel 2: Expression von thpD in Streptomyces lividans

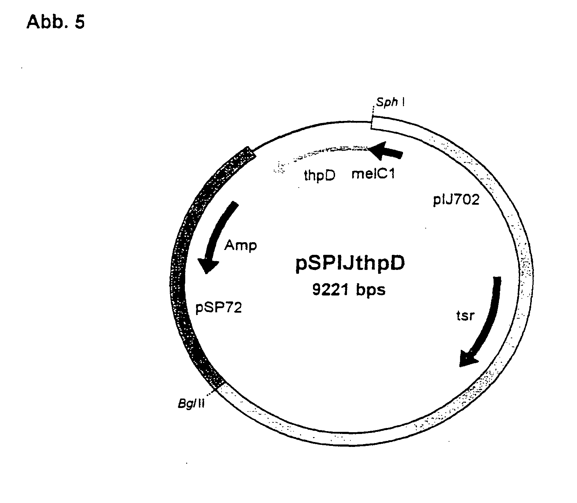

Zur Expression von *thpD in Streptomyces lividans* wurde das Gen *thpD als SphI*/*HindIII* Fragment in den Expressionsvektor pSPIJ002 (Abb. 5) kloniert. Das resultierende Expressionsplasmid wurde als pSPIJthpD bezeichnet. Durch PCR Mutagenese wurden die Restriktionsschnittstellen *SphI und HindIII* eingeführt (35 Zyklen; 1 min 95°C; 90 sec 55°C; 65 sec 72°C; Primer siehe Tabelle 1). Als Matrize für die PCR diente das Plasmid pQE30thpD. Bei diesem Plasmid handelt es sich um ein pQE30 Derivat, welches das *thpD* Gen als *BamHI*/*HindIII* PCR-Fragment enthält (35 Zyklen; 1 min 95°C; 90 sec 55°C; 65 sec 72°C; Primer siehe Tab.1). Die verwendeten Primer sind in Tabelle 1 als SEQ. ID. NO.: 2-5 gezeigt. Die Ligation und Transformation erfolgte nach Standardmethoden.

### Beispiel 3: Reinigung von Hexa-His-THPD aus Streptomyces lividans

Von einer Vorkultur des *Streptomyces lividans* Stammes (transformiert mit pSPIJthpD, siehe Beispiel 2) wurde YEME-Medium (340 g/l Sucrose, 10 g/l Glucose, 3 g/l Hefeextrakt, 3 g/l Malzextrakt, 5 g/l Bacto-Pepton und 0,2 % MgCl₂) auf eine OD₆₀₀ von 0.05 angeimpft und im Schüttler bei 28°C inkubiert. Nach 72 Stunden wurden die Zellen geerntet. Die Zellen wurden in Aufschlußpuffer (100 mM Phosphatpuffer pH 8.0, 10% Glycerol, 1 mM Benzamidin, 1 mM PMSF, 10 mM Imidazol und 300 mM NaCl) resuspendiert und durch zweimalige Passage durch eine French-Press-Cell (16.000 psi; entspricht 1,105x10⁸ Pa) aufgeschlossen. Der zellfreie Rohextrakt wurde im SS34-Rotor 30 min bei 12000 upm zentrifugiert. Der zellfreie Überstand wurde an einer Ni-NTA-Matrix chromatographiert. Das Hexa-His-THPD Protein eluiert im Bereich von 30-100 mM Imidazol.

### Beispiel 4: In vitro Synthese von 2-Methyl-4-carboxy-5-hydroxy-3,4,5,6-tetrahydropyrimidin (THP(A))

Die Bestimmung der THP(B)-Dioxygenase-Aktivität erfolgt unter Verwendung des folgenden Reaktionsansatzes:
Kaliumphosphatpuffer, pH 8 10 mM
THP(B) 5 mM
α-Ketoglutarat 5 mM
Ascorbinsäure 5 mM
Eisen(II)sulfat 1 mM
Hexa-His-THPD variabel
in einem Gesamtvolumen von 100 µl.

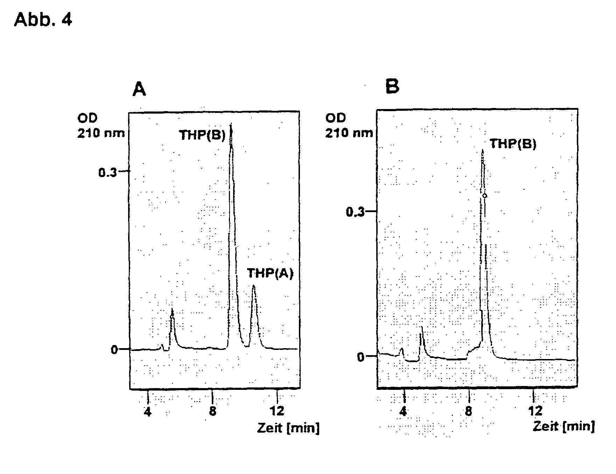

Die Inkubation erfolgt bei 30°C für eine Stunde. Die Produktanalyse erfolgt durch Chromatographie an einer NH₂-Nucleosil-HPLC-Säule. Die Elution erfolgte mit 70% Acetonitril/H₂O. Das Elutionsprofil ist in Abb. 4 dargestellt. Unter Verwendung von Katalase (Rinderleber, Fa. Sigma) kann eine vollständige Hydroxylierung von THP(B) zu THP(A) erreicht werden.
Abb. 1: Biosynthese von THP(B) ausgehend von L-Aspartat-β-Semialdehyd. A: L-2,4-Diaminobuttersäure Transaminase, B: L-2,4-Diaminobuttersäure-Acetyltransferase, C: N-Acetyl-Diaminobuttersäure-Cyclase.
Abb. 2: Restriktionskarte des sequenzierten Bereichs sowie die Lage der aufgefundenen THP-Biosynthesegene. Unter den Restriktionsenzymen ist die entsprechende Basenposition angegeben.
Abb. 3: Interne tryptische Peptidsequenz der DABA-Acetyltransferase *aus Streptomyces chrysomallus* und davon abgeleitete Oligonukleotidsequenzen (170498A und 170498B).
Abb. 4: HPLC von THP(A) und THP(B) an einer NH₂-Nucleosil-Säule. Jeweils 1/10 des Reaktionsansatzes, der 5 mM THP(B), 5 mM α-Ketoglutarat, 5 mM Ascorbinsäure, 1 mM FeSO₄ und 2 mg/ml THPD-Fusionsprotein (A) bzw. Puffer D (B) enthielt, wurde aufgetragen. Die Elution erfolgte mit 70% Acetonitril bei einer Flußrate von 1 ml/min.
Abb. 5: pSPIJOO2: Shuttle-Vektor, der durch Ligation von pSP72 (Bglll) und plJ702 (Bglll) entstanden ist. Dieser Expressionsvektor kann sowohl in E. coli als auch in Streptomyces verwendet werden.

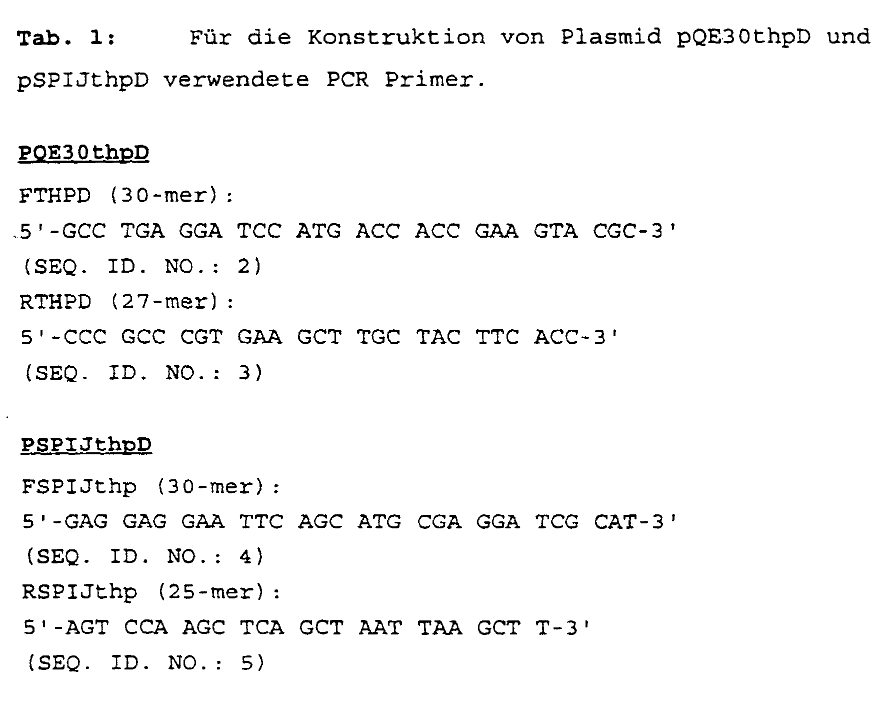

## Patentansprüche

1. Nukleinsäuresequenz, wobei die Nukleinsäuresequenz für ein Polypeptid mit Tetrahydropyrimidin-Dioxygenase-Aktivität kodiert und aus der folgenden Gruppe von Nukleinsäuresequenzen ausgewählt ist:
(a) DNA-Nukleinsäuresequenz mit der in SEQ. ID. NO.: 1 dargestellten Nukleotidsequenz;
(b) DNA-Nukleinsäuresequenz, die sich als Ergebnis des degenerierten genetischen Codes von der in SEQ. ID. NO.: 1 dargestellten Nukleotidsequenz ableitet;
(c) DNA-Nukleinsäuresequenz, die mit mindestens einer DNA-Nukleinsäuresequenz, die eine DNA-Nukleinsäuresequenz gemäß (a) oder (b) aufweist, hybridisiert;
(d) DNA-Nukleinsäuresequenz, die Fragmente, allelische oder andere Variationen der DNA-Nukleinsäuresequenz mit der in SEQ. ID. NO.: 1 dargestellten Nukleotidsequenz, ist;
(e) RNA-Nukleinsäuresequenz, die von einer DNA-Nukleinsäuresequenz gemäß (a) bis (d) abgeleitet ist; und
(f) Nukleinsäuresequenz mit einem Grad der Homologie zu einer der Sequenzen gemäß (a) bis (e) von mehr als 60%.

2. Nukleinsäuresequenz gemäß Anspruch 1, wobei die Nukleinsäuresequenz eine genomische DNA-Nukleinsäuresequenz oder eine cDNA-Nukleinsäuresequenz ist.

3. Nukleinsäuresequenz gemäß einem der Ansprüche 1 oder 2, wobei die Nukleinsäuresequenz aus einem Archaebakterium, einem Prokaryonten oder einem Eukaryonten isoliert wurde.

4. Nukleinsäuresequenz gemäß Anspruch 3, wobei die Nukleinsäuresequenz aus einem halophilen Eubakterium, einem Actinomyceten, einem Bazillus oder einem Brevibakterium isoliert wurde.

5. Nukleinsäurekonstrukt, wobei das Nukleinsäurekonstrukt in einer operativen Einheit eine Promotersequenz und eine Nukleinsäuresequenz gemäß einem der Ansprüche 1 oder 2 umfasst.

6. Nukleinsäurekonstrukt gemäß Anspruch 5, wobei das Nukleinsäurekonstrukt zusätzlich eine Nukleinsäuresequenz umfasst, die für ein oder mehrere Sekretionssignale kodiert.

7. Polypeptid mit Tetrahydropyrimidin-Dioxygenase-Aktivität, wobei das Polypeptid durch eine Nukleinsäuresequenz gemäß einem der Ansprüche 1 oder 2 kodiert wird.

8. Polypeptid nach Anspruch 7, wobei das Polypeptid die Aminosäuresequenz SEQ. ID. NO.: 6 hat.

9. Replikativer, rekombinanter Vektor, wobei der Vektor eine Nukleinsäuresequenz gemäß einem der Ansprüche 1 oder 2 oder ein Nukleinsäurekonstrukt gemäß einem der Ansprüche 5 oder 6 umfasst.

10. Zelle, wobei die Zelle einen Vektor gemäß Anspruch 9 enthält.

11. Zelle, wobei die Zelle eine durch nicht-natürliche Rekombination in das Genom integrierte Nukleinsäuresequenz gemäß einem der Ansprüche 1 oder 2 aufweist.

12. Zelle gemäß Anspruch 10 oder 11, wobei die Zelle eine Bakterienzelle, eine Hefezelle oder eine Pflanzenzelle ist.

13. Pflanze, die mit einer rekombinanten DNA, die eine DNA-Sequenz gemäß einem der Ansprüche 1 bis 4 umfasst, transformiert wurde.

14. Verwendung von einem Oligonukleotid, das von einer Nukleinsäuresequenz gemäß einem der Ansprüche 1 oder 2 abgeleitet ist, als Nukleinsäure-Sonde zur Identifizierung von chromosomalen oder extrachromosomalen, sich in Archaebakterien, Prokaryonten oder Eukaryonten befindlichen Genen, die für ein Polypeptid kodieren, welches Tetrahydropyrimidin-Dioxygenase-Aktivität besitzt.

15. Verwendung von einem Polypeptid mit Tetrahydropyrimidin-Dioxygenase-Aktivität zur Herstellung von hydroxyliertem Tetrahydropyrimidin, wobei das Polypeptid durch eine Nukleinsäuresequenz gemäß Anspruch 1 kodiert wird.

16. Verwendung nach Anspruch 15, wobei das Polypeptid die Aminosäuresequenz SEQ. ID. NO.: 6 hat.

17. Verwendung von einer Zelle nach einem der Ansprüche 10-12 zur Herstellung von hydroxyliertem Tetrahydropyrimidin.

18. Verfahren zur Herstellung eines Polypeptids mit Tetrahydropyrimidin-Dioxygenase-Aktivität, wobei
Zellen gemäß einem der Ansprüche 10 bis 12, wobei die Zellen das Polypeptid mit Tetrahydropyrimidin-Dioxygenase-Aktivität exprimieren, in einem Kulturmedium kultiviert werden; und
das Polypeptid mit Tetrahydropyrimidin-Dioxygenase-Aktivität anschließend isoliert wird.

19. Verfahren gemäß Anspruch 18, wobei die lebende Zelle permeabilisiert worden ist und Tetrahydropyrimidin im Kulturmedium enthalten ist.

## Claims

1. Nucleic acid sequence, wherein the nucleic acid sequence codes for a polypeptide having tetrahydropyrimidine dioxygenase activity and is selected from the following group of nucleic acid sequences:
(a) DNA nucleic acid sequence having the nucleotide sequence shown in SEQ. ID. No.: 1;
(b) DNA nucleic acid sequence derived from the nucleotide sequence shown in SEQ. ID. No.: 1 as a result of the degenerate genetic code;
(c) DNA nucleic acid sequence which hybridises with at least one DNA nucleic acid sequence having a DNA nucleic acid sequence according to (a) or (b);
(d) DNA nucleic acid sequence which is fragments, allelic or other variations of the DNA nucleic acid sequence having the nucleotide sequence shown in SEQ. ID. No.: 1;
(e) RNA nucleic acid sequence derived from a DNA nucleic acid sequence according to (a) to (d); and
(f) nucleic acid sequence having a degree of homology with one of the sequences according to (a) to (e) of more than 60 %.

2. Nucleic acid sequence according to claim 1, wherein the nucleic acid sequence is a genomic DNA nucleic acid sequence or a cDNA nucleic acid sequence.

3. Nucleic acid sequence according to either claim 1 or claim 2, wherein the nucleic acid sequence has been isolated from an archaebacterium, a prokaryote or a eukaryote.

4. Nucleic acid sequence according to claim 3, wherein the nucleic acid sequence has been isolated from a halophilic eubacterium, an actinomycete, a bacillus or a brevibacterium.

5. Nucleic acid construct, wherein the nucleic acid construct comprises in an operative unit a promoter sequence and a nucleic acid sequence according to either claim 1 or claim 2.

6. Nucleic acid construct according to claim 5, wherein the nucleic acid construct additionally comprises a nucleic acid sequence that codes for one or more secretion signals.

7. Polypeptide having tetrahydropyrimidine dioxygenase activity, wherein the polypeptide is encoded by a nucleic acid sequence according to either claim 1 or claim 2.

8. Polypeptide according to claim 7, wherein the polypeptide has the amino acid sequence SEQ. ID. No.: 6.

9. Replicative, recombinant vector, wherein the vector comprises a nucleic acid sequence according to either claim 1 or claim 2 or a nucleic acid construct according to either claim 5 or claim 6.

10. Cell, wherein the cell contains a vector according to claim 9.

11. Cell, wherein the cell has a nucleic acid sequence according to either claim 1 or claim 2 integrated into the genome by non-natural recombination.

12. Cell according to claim 10 or claim 11, wherein the cell is a bacterial cell, a yeast cell or a plant cell.

13. Plant transformed with a recombinant DNA comprising a DNA sequence according to any one of claims 1 to 4.

14. Use of an oligonucleotide derived from a nucleic acid sequence according to either claim 1 or claim 2 as a nucleic acid probe for the identification of chromosomal or extra-chromosomal genes which are present in archaebacteria, prokaryotes or eukaryotes and that code for a polypeptide having tetrahydropyrimidine dioxygenase activity.

15. Use of a polypeptide having tetrahydropyrimidine dioxygenase activity for the production of hydroxylated tetrahydropyrimidine, wherein the polypeptide is encoded by a nucleic acid sequence according to claim 1.

16. Use according to claim 15, wherein the polypeptide has the amino acid sequence SEQ. ID. No.: 6.

17. Use of a cell according to any one of claims 10 to 12 for the production of hydroxylated tetrahydropyrimidine.

18. Method for the production of a polypeptide having tetrahydropyrimidine dioxygenase activity, wherein
cells according to any one of claims 10 to 12, wherein the cells express the polypeptide having tetrahydropyrimidine dioxygenase activity, are cultured in a culture medium; and
the polypeptide having tetrahydropyrimidine dioxygenase activity is subsequently isolated.

19. Method according to claim 18, wherein the living cell has been permeabilised and the culture medium contains tetrahydropyrimidine.

## Revendications

1. Séquence d'acide nucléique, où la séquence d'acide nucléique code pour un polypeptide avec activité de tétrahydropyrimidine dioxygénase et est choisie parmi le groupe suivant de séquences d'acide nucléique :
(a) une séquence d'acide nucléique ADN avec la séquence de nucléotides représentée en SEQ ID N°1 ;
(b) une séquence d'acide nucléique ADN, qui dérive de la séquence de nucléotides représentée en SEQ ID N°1 suite au code génétique dégénéré ;
(c) une séquence d'acide nucléique ADN, qui hybride avec au moins une séquence d'acide nucléique ADN, qui présente une séquence d'acide nucléique ADN selon (a) ou (b) ;
(d) une séquence d'acide nucléique ADN, les fragments, variants alléliques ou autres de la séquence d'acide nucléique ADN, avec la séquence de nucléotides représentée en SEQ ID N°1 ;
(e) une séquence d'acide nucléique ARN, qui dérive d'une séquence d'acide nucléique ADN selon (a) à (d), et
(f) une séquence d'acide nucléique avec un taux d'homologie de plus de 60% avec une des séquences selon (a) à (e).

2. Séquence d'acide nucléique selon la revendication 1, où la séquence d'acide nucléique est une séquence d'acide nucléique ADN génomique ou une séquence d'acide nucléique ADNc.

3. Séquence d'acide nucléique selon la revendication 1 ou 2, où la séquence d'acide nucléique est isolée d'une Archacbactérie, d'un procaryote ou d'un eucaryote.

4. Séquence d'acide nucléique selon la revendication 3, où la séquence d'acide nucléique est isolée d'une Eubactérie halophile, d'un Actinomycète, d'un Bacillus ou d'une Brevibactérie.

5. Construction d'acide nucléique, où la construction d'acide nucléique comprend dans une unité fonctionnelle, une séquence promoteur et une séquence d'acide nucléique selon une des revendications 1 ou 2.

6. Construction d'acide nucléique selon la revendication 5, où la construction d'acide nucléique comprend en outre, une séquence d'acide nucléique, qui code pour un ou plusieurs signaux de sécrétion.

7. Polypeptide avec activité de tétrahydropyrimidine dioxygénase, où le polypeptide est codé par une séquence d'acide nucléique selon une des revendications 1 ou 2.

8. Polypeptide selon la revendication 7, où le polypeptide a la séquence des acides aminés SEQ ID N°6.

9. Vecteur recombiné de réplication, où le vecteur comprend une séquence d'acide nucléique selon une des revendications 1 ou 2 ou une construction d'acide nucléique selon une des revendications 5 ou 6.

10. Cellule, où la cellule contient un vecteur selon la revendication 9.

11. Cellule, où la cellule présente une séquence d'acide nucléique selon une des revendications 1 ou 2, intégrée dans le génome par recombinaison non naturelle.

12. Cellule selon la revendication 10 ou 11, où la cellule est une cellule bactérienne, une cellule de levure ou une cellule végétale.

13. Plante, qui est transformée avec un ADN recombiné, qui comprend une séquence d'ADN selon une des revendications 1 à 4.

14. Utilisation d'un oligonucléotide, qui dérive d'une séquence d'acide nucléique selon une des revendications 1 ou 2, comme sonde acide nucléique pour l'identification de gènes chromosomiques ou extrachromosomiques, se trouvant DANS des Archacbactéries, procaryotes ou eucaryotes, qui codent pour un polypeptide, qui possède l'activité de tétrahydropyrimidine dioxygénase.

15. Utilisation d'un polypeptide avec activité de tétrahydropyrimidine dioxygénase pour la préparation de tétrahydropyrimidine hydroxylée, où le polypeptide est codé par une séquence d'acide nucléique selon la revendication 1.

16. Utilisation selon la revendication 15, où le polypeptide a la séquence des acides aminés SEQ ID N°6.

17. Utilisation d'une cellule selon l'une des revendications 10 à 12, pour la préparation de tétrahydropyrimidine hydroxylée.

18. Procédé de préparation d'un polypeptide avec activité de tétrahydropyrimidine dioxygénase, où des cellules selon l'une des revendications 10 à 12 sont mises en culture dans un milieu de culture, les cellules exprimant le polypeptide avec activité de tétrahydropyrimidine dioxygénase, et
le polypeptide avec activité de tétrahydropyrimidine dioxygénase est ensuite isolé.

19. Procédé selon la revendication 18, où la cellule vivante est rendue perméable et où la tétrahydropyrimidine est présente dans le milieu de culture.
